(19) Europäisches Patentamt
European Patent Office
Office européen des brevets

(11) **EP 3 248 606 B1**

(12) **EUROPEAN PATENT SPECIFICATION**

(45) Date of publication and mention
of the grant of the patent:
**19.11.2025 Bulletin 2025/47**

(21) Application number: **17172420.6**

(22) Date of filing: **23.05.2017**

(51) International Patent Classification (IPC):
*A61K 36/48* (2006.01)  *A61K 36/235* (2006.01)
*A61K 36/185* (2006.01)

(52) Cooperative Patent Classification (CPC):
(C-Sets available)
**C12N 9/2497; A61K 36/185; A61K 36/235;
A61K 36/48; A61P 3/10; A61P 9/10; A61P 17/18;
A61P 25/28; A61P 27/12;** C07H 21/04; C12N 9/00;
C12N 9/14; C12Y 302/01                      (Cont.)

(54) **OXIDIZED PROTEIN HYDROLASE ACTIVITY ENHANCING AGENT AND GLYCATION STRESS INHIBITOR**

OXIDIERTE PROTEINHYDROLASEAKTIVITÄT ERHÖHENDES MITTEL UND GLYKIERUNGSSTRESSINHIBITOR

AGENT D'AMÉLIORATION DE L'ACTIVITÉ HYDROLASE DES PROTÉINES OXYDÉES ET INHIBITEUR DU STRESS DE GLYCATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.05.2016 JP 2016102697
23.05.2016 JP 2016102702
22.05.2017 JP 2017100806**

(43) Date of publication of application:
**29.11.2017 Bulletin 2017/48**

(73) Proprietor: **ARKRAY, Inc.
Minami-ku
Kyoto-shi,
Kyoto 601-8045 (JP)**

(72) Inventors:
• **Shoshihara, Masako
Kyoto, 602-0008 (JP)**
• **Kawai, Hiroshige
Kyoto, 602-0008 (JP)**

(74) Representative: **Hoffmann Eitle
Patent- und Rechtsanwälte PartmbB
Arabellastraße 30
81925 München (DE)**

(56) References cited:
EP-A1- 1 683 805      EP-A1- 2 949 362
JP-A- H11 106 336

• **EDWIN JARALD ET AL: "Diabetes and herbal medicines", IRANIAN JOURNAL OF PHARMACOLOGY AND THERAPEUTICS, IRAN UNIVERSITY OF MEDICAL SCIENCES RAZI INSTITUTE FOR DRUG RESEARCH, IRAN, ISLAMIC REPUBLIC OF, vol. 7, no. 1, 1 January 2008 (2008-01-01), pages 97 - 106, XP002656225, ISSN: 1735-2657**
• **DATABASE BIOSIS [online] BIOSCIENCES INFORMATION SERVICE, PHILADELPHIA, PA, US; January 2012 (2012-01-01), RASUL A ET AL: "Formulation development of a cream containing fennel extract: in vivo evaluation for anti-aging effects", XP002774647, Database accession no. PREV201200211363**
• **PHARMAZIE, vol. 67, no. 1, January 2012 (2012-01-01), pages 54 - 58, ISSN: 0031-7144, DOI: 10.1691/PH.2012.1083**
• **GURROLA-DIAZ C M ET AL: "Effects of Hibiscus sabdariffa extract powder and preventive treatment (diet) on the lipid profiles of patients with metabolic syndrome (MeSy)", PHYTOMEDICINE, ELSEVIER, AMSTERDAM, NL, vol. 17, no. 7, 1 June 2010 (2010-06-01), pages 500 - 505, XP027012957, ISSN: 0944-7113, [retrieved on 20091203]**

• **RASUL A ET AL: "Formulation development of a cream containing fennel extract: in vivo evaluation for anti-aging effects", PHARMAZIE, vol. 67, no. 1, January 2012 (2012-01-01), pages 54 - 58, ISSN: 0031-7144(print)**
• **DATABASE BIOSIS [online] BIOSCIENCES INFORMATION SERVICE, PHILADELPHIA, PA, US; January 2012 (2012-01-01), RASUL A ET AL: "Formulation development of a cream containing fennel extract: in vivo evaluation for anti-aging effects", XP002774647, Database accession no. PREV201200211363**

• **PHARMAZIE, vol. 67, no. 1, January 2012 (2012-01-01), pages 54 - 58, ISSN: 0031-7144(print), DOI: 10.1691/PH.2012.1083**

(52) Cooperative Patent Classification (CPC): (Cont.)

C-Sets
**A61K 36/185, A61K 2300/00;
A61K 36/235, A61K 2300/00;
A61K 36/48, A61K 2300/00**

• **RASUL A ET AL: "Formulation development of a cream containing fennel extract: in vivo evaluation for anti-aging effects", PHARMAZIE, vol. 67, no. 1, January 2012 (2012-01-01), pages 54 - 58, ISSN: 0031-7144(print)**

• **PHARMAZIE, vol. 67, no. 1, January 2012 (2012-01-01), pages 54 - 58, ISSN: 0031-7144(print), DOI: 10.1691/PH.2012.1083**

**Description**

BACKGROUND OF THE INVENTION

1. Field of the Invention

The present invention relates to the non-therapeutic use of an agent, comprising an extract of fenugreek as an active ingredient, for improving the efficiency of decomposing oxidized proteins and glycated proteins as a result of enhancing oxidized protein hydrolase activity as defined in the claims.

2. Description of Related Art

It has been known that oxidation reactions and glycation reactions in a living body adversely affect cells and tissues. Oxidized proteins and glycated proteins cause, by production and accumulation thereof, diseases such as diabetic complications, Alzheimer's disease, cataract, and arteriosclerosis as well as aging and functional decline of tissues such as the skin. It has been known that generally, these proteins are decomposed and removed by proteolytic enzymes such as oxidized protein hydrolase (OPH) and proteasome but the activities of these enzymes are reduced with aging.

As substances for enhancing the OPH activity, extracts of plants such as *Anthemis nobilis, Houttuynia cordata, Crataegus oxyacantha, Vitis vinifera,* and *Trapa japonica* have been proposed (WO2011/004733, and WO2014/126199).

The "glycation stress" is a concept that comprehensively includes the stress put on a living body by reducing sugar or aldehyde stress and the reaction thereafter. Abnormal proteins produced by glycation stress have been attracting attention as being related to diseases such as lifestyle-related diseases and aging.

Generally, the abnormal proteins are proteins subjected to oxidation, glycation, or aldehyde modification due to aging, etc. It has been considered that the accumulation thereof in a living body causes functional decline of functional proteins, inflammation, etc., which results in diseases such as diabetic complications, Alzheimer's disease, cataract, and arteriosclerosis as well as aging and functional decline of tissues such as the skin.

The Maillard reaction in a living body has been considered as one of the causes of the abnormal proteins being produced. For example, a glycation reaction (the Maillard reaction) in a living body subjects proteins such as collagen to glycation to change amino acids contained in the proteins into AGEs (advanced glycation end products), or AGEs cause cross-linked structures to be formed between proteins, which results in abnormal proteins.

N-phenacylthiazolium bromide (PTB) and N-phenacyl-4,5-dimethylthiazolium bromide (ALT-711) are known as drugs that decompose the cross-linked structures formed between proteins by AGEs (Sara Vasan et al., Nature, 382, p275-278 (1996), and Sara Vasan et al., Archives of Biochemistry and Biophysics, 419, p89-96 (2003)). However, they are not used in terms of stability and side effects. In addition, for example, a lotus leaf extract and an *Astilbe thunbergii* extract have been studied (JP2007-119373A). Further, different extracts including peanut, rosemary, knotweed, leaf lettuce, basil, thyme, spearmint, lemon balm, perilla und rhubarb have been disclosed for the decomposition of AGEs (EP 2 949 362 A1).

SUMMARY OF THE INVENTION

AGEs and ALEs (advanced lip oxidation end products) are produced by the reaction (the Maillard reaction) between protein or lipid in a living body and a reactive group such as an aldehyde group or a ketone group formed through, for example, β-oxidation or peroxidation of lipid or reducing sugar such as glucose. It is becoming clear that these are produced and accumulated in a living body to cause, for example, dysfunction of functional proteins and activation of receptors for AGEs. These are considered as one of the factors that cause, for example, hypoactivity, inflammation, lifestyle-related diseases, and aging, and the concept that comprehensively includes a series of these phenomena and symptoms is referred to as glycation stress or carbonyl stress.

Furthermore, it is considered that when functional proteins are subjected to glycation by the Maillard reaction, which causes a dysfunction, active oxygen is generated excessively or an active oxygen eliminating action in a living body stops fully functioning, which results in oxidative stress. As is the case with glycation stress, oxidative stress is also considered as one of the causes of aging and various diseases including lifestyle-related diseases.

In terms of, for example, anti-aging and preventing lifestyle-related diseases, there are demands for new substances and methods that can inhibit, decompose, or remove the above-mentioned abnormal proteins to inhibit or reduce glycation stress and oxidative stress.

Moreover, there are expectations to possibly treat and prevent the above-mentioned diseases, the above-mentioned aging of tissues, and the above-mentioned functional decline of tissues by enhancing the activity of enzymes such as OPH.

Thus, in an aspect, the present disclosure provides a new substance that can enhance OPH activity and a new substance that can inhibit or reduce glycation stress.

The present invention relates to non-therapeutic use of an oxidized protein hydrolase activity enhancing agent containing an extract of fenugreek as an active ingredient as defined in the claims.

As another aspect, the present invention relates to a glycation stress inhibitor containing extracts of fenugreek and fennel as active ingredients as defined in the claims.

As another aspect, the present invention relates to the non-therapeutic use of an oxidized protein hydrolase activity enhancing agent or a glycation stress inhibitor, wherein the oxidized protein hydrolase activity enhancing agent or the glycation stress inhibitor is comprised as an active agent in a beverage composition, a food composition, a functional food, a cosmetic, or a supplement for inhibiting glycation stress, each of which contains extracts of fenugreek and fennel as active ingredients as defined in the claims.

DETAILED DESCRIPTION OF THE INVENTION

[OPH activity enhancing agent]

The present invention is based on a new finding that water extracts of fenugreek (scientific name: *Trigonella foenum -graecum*), fennel (scientific name: *Foeniculum vulgare),* and hibiscus (scientific name: *Hibiscus sabdariffa)* can enhance the activity of an Acylamino-acid-releasing enzyme (AARE) known as OPH.

OPH is known to be widely distributed in a living body and to preferentially decompose substances resulting from glycation stress or oxidative stress in a living body, such as oxidized proteins and glycated proteins. Therefore, enhancing the enzyme activity of OPH can improve the efficiency of decomposing oxidized proteins and glycated proteins, and thereby it can be expected that glycation stress or oxidative stress can be inhibited or reduced. Since the OPH activity enhancing agent of the present disclosure can enhance the enzyme activity of OPH, oxidized proteins can be decomposed and removed or the accumulation of oxidized proteins can be inhibited in one or more embodiments. In one or more embodiments, the OPH activity enhancing agent of the present disclosure can inhibit or reduce glycation stress and/or oxidative stress. In one or more embodiments, the OPH activity enhancing agent of the present invention can prevent, improve, or treat diseases caused by glycation stress or oxidative stress, aging of tissues, and functional decline of tissues. In one or more embodiments, the OPH activity enhancing agent of the present invention can improve metabolism.

In an aspect, the present invention relates to the non-therapeutic use of an OPH activity enhancing agent containing an extract of fenugreek as an active ingredient as defined in the claims. In one or more embodiments, the OPH activity enhancing agent of the present invention may further contain an extract of fennel. In one or more embodiments, the OPH activity enhancing agent of the present invention may further contain an extract of hibiscus. In one or more embodiments, the OPH activity enhancing agent of the present invention contains, as active ingredients, an extract of fenugreek and at least one of extracts of fennel and hibiscus. Furthermore, in another aspect, the present invention relates to an OPH activity enhancing agent containing, as active ingredients, extracts of fenugreek, fennel, and hibiscus.

When the OPH activity enhancing agent of the present invention contains an extract of another plant in addition to fenugreek, they may be blended at the same ratio (1 part by weight with respect to 1 part by weight of the extract of fenugreek) or they may be blended at different ratios.

In one or more embodiments, the OPH activity enhancing agent of the present invention contains an extract of fenugreek and an extract of fennel. In the present aspect, the blending ratio (the dry weight ratio) of the extract of fenugreek and the extract of fennel is, per 1 part by weight of the extract of fenugreek, 0.1 to 10 parts by weight of the extract of fennel, and 0.2 to 5 parts by weight of the extract of fennel in terms of enhancing the OPH activity.

In one or more embodiments, the OPH activity enhancing agent of the present invention contains an extract of fenugreek and an extract of hibiscus. In the present aspect, the blending ratio (the dry weight ratio) of the extract of fenugreek and the extract of hibiscus is, per 1 part by weight of the extract of fenugreek, 0.1 to 10 parts by weight of the extract of hibiscus, and 0.2 to 5 parts by weight of the extract of hibiscus in terms of enhancing the OPH activity.

In one or more embodiments, the OPH activity enhancing agent of the present invention contains an extract of fenugreek, an extract of fennel, and an extract of hibiscus. In the present aspect, the blending ratio (the dry weight ratio) of the extract of fenugreek, the extract of fennel, and the extract of hibiscus is, per 1 part by weight of the extract of fenugreek, 0.05 to 20 parts by weight of the extract of fennel and 0.05 to 20 parts by weight of the extract of hibiscus, and 0.1 to 10 parts by weight of the extract of fennel and 0.1 to 10 parts by weight of the extract of hibiscus in terms of enhancing the OPH activity.

The extracts may be extracted from any parts of plants. In one or more embodiments, examples of the part to be used include whole plants, flowers, leaves, seeds, fruits, branches, stems, roots, calyxes, and bracts. The part to be used may be of one type, or two or more types of parts may be combined for use. In one or more embodiments, fenugreek is preferably extracted from seeds. In one or more embodiments, fennel is preferably extracted from seeds. In one or more embodiments, hibiscus is preferably extracted from calyxes and bracts.

The extraction method is not particularly limited, but in one or more embodiments, examples thereof include solvent extraction. In one or more embodiments, examples of the extractant (solvent) include water, alcohols such as methanol and ethanol, and ether. In one or more embodiments, examples of the extract include a water extract and an organic solvent extract. In one or more embodiments, examples of the water extract include a hot water extract. In one embodiment, the solvent extracting is performed at a temperature of 60-120°C, preferably 70-90°C such as 80°C or 100°C, for a time period of 15 minutes to 5 hours, preferably 2-6 hours or 3-5 hours such as 30 minutes or 1 hour. In a preferred embodiment, hot water extraction is carried out at a temperature of 60-120°C, preferably 70-90°C such as 80°C or 100°C, for a time period of 30 minutes to 5 hours, preferably 2-6 hours or 3-5 hours such as 30 minutes or 1 hour. In one or more embodiments, examples of the form of the extract include an aqueous solution, a concentrated liquid, and a dried product. Drying is carried out by heat-treating the extract obtained by the solvent-extracting at a temperature of 70-150°C such as 110°C for 1-10 hours such as 4 hours. A suitable method for producing an OPH activity enhancing agent according to the present invention is as follows: A defined amount of dry powder of fenugreek (such as 2 g) and a defined amount of dry powder of fennel (such as 2 g) are extracted with a suitable amount of distilled water (such as 40 mL) at a temperature of 80°C for one hour, followed by cooling to normal temperature. Thereafter, the obtained slurry is filtrated and the filtrate thus obtained is used as a plant extract. If a dried plant extract shall be used, the obtained plant extract is put into an aluminum tray and dried for e.g. four hours in an incubator set at e.g. 110°C.

The form of the OPH activity enhancing agent of the present invention is not particularly limited, but in one or more embodiments, examples thereof include solids, granules, powders, pastes, and liquids.

In one or more embodiments, the OPH activity enhancing agent of the present invention may contain extracts of other foods and other components as long as they do not inhibit the OPH activity enhancing functions of the active ingredients.

In one or more embodiments, the OPH activity enhancing agent of the present invention can be non-therapeutically used as a raw material for a beverage composition, a food composition, a functional food, a cosmetic, or a supplement. The form of the raw material is not particularly limited, but in one or more embodiments, examples thereof include solids, granules, powders, pastes, and liquids. As one or more embodiments, the present invention relates to the non-therapeutic use of the OPH activity enhancing agent, wherein the oxidized protein hydrolase activity enhancing agent is comprised as an active agent in a beverage composition, a food composition, a functional food, a cosmetic, or a supplement that contains an OPH activity enhancing agent of the present invention.

The intake per day of the OPH activity enhancing agent of the present invention is not particularly limited, but in one or more embodiments, it is at least 5 mg or not more than 2000 mg.

[Method of producing OPH activity enhancing agent]

In another aspect, the present disclosure relates to a method of producing an OPH activity enhancing agent for enhancing OPH activity (a method of producing an OPH activity enhancing agent of the present disclosure). In one or more embodiments, the method of producing an OPH activity enhancing agent of the present disclosure includes extracting fenugreek with a solvent. In one or more embodiments, the method of producing an OPH activity enhancing agent of the present disclosure may further include extracting one or more types of plants selected from the group consisting of fennel and hibiscus with a solvent.

Furthermore, in one or more embodiments, the method of producing an OPH activity enhancing agent of the present disclosure relates to a method of producing an OPH activity enhancing agent for enhancing OPH activity, the method including extracting one or more types of plants selected from the group consisting of fenugreek, fennel, and hibiscus with a solvent. According to the method of producing an OPH activity enhancing agent of the present disclosure, the OPH activity enhancing agent of the present disclosure can be produced.

In one or more embodiments, the solvent-extracting may include solvent-extracting a dried plant.

In one or more embodiments, the method of producing an OPH activity enhancing agent of the present disclosure may include filtrating or centrifuging an extract obtained by solvent-extracting.

In one or more embodiments, the method of producing an OPH activity enhancing agent of the present disclosure may include drying a resultant extract.

In one or more embodiments, the production method of the present disclosure may include mixing an extract of fenugreek and an extract of a plant other than fenugreek to obtain a mixture of the extracts. In one or more embodiments, the mixing ratios thereof may be the same (1 part by weight with respect to 1 part by weight of the extract of fenugreek) or may be different from each other.

In one or more embodiments, the production method of the present disclosure may include mixing an extract of fenugreek and an extract of fennel so that the extract of fennel is 0.1 to 10 parts by weight per 1 part by weight of the extract of fenugreek, and in terms of producing an OPH activity enhancing agent that can further enhance the OPH activity, the production method of the present disclosure may include mixing these extracts so that the extract of fennel is 0.2 to 5 parts by weight per 1 part by weight of the extract of fenugreek.

In one or more embodiments, the production method of the present disclosure may include mixing an extract of fenugreek and an extract of hibiscus so that the extract of hibiscus is 0.1 to 10 parts by weight per 1 part by weight of the extract of fenugreek, and in terms of producing an OPH activity enhancing agent that can further enhance the OPH activity, the production method of the present disclosure may include mixing these extracts so that the extract of hibiscus is 0.2 to 5 parts by weight per 1 part by weight of the extract of fenugreek.

In one or more embodiments, the production method of the present disclosure may include mixing an extract of fenugreek, an extract of fennel, and an extract of hibiscus so that, per 1 part by weight of the extract of fenugreek, the extract of fennel is 0.05 to 20 parts by weight and the extract of hibiscus is 0.05 to 20 parts by weight, and in terms of producing an OPH activity enhancing agent that can further enhance the OPH activity, the production method of the present disclosure may include mixing these extracts so that, per 1 part by weight of the extract of fenugreek, the extract of fennel is 0.1 to 10 parts by weight and the extract of hibiscus is 0.1 to 10 parts by weight.

In one or more embodiments, the production method of the present disclosure may include mixing fenugreek and a plant other than fenugreek and solvent-extracting the resultant mixture to obtain a mixed extract. In one or more embodiments, the mixing ratios thereof may be the same (1 part by weight with respect to 1 part by weight of the fenugreek) or may be different from each other. Furthermore, they may be mixed together so that the ratios of the extracts of the respective plants in the resultant mixed extract are the same (1 part by weight with respect to 1 part by weight of the extract of fenugreek) or they may be mixed together so that the ratios thereof are different from each other.

[Method of enhancing OPH activity]

In an aspect, the present disclosure relates to a method of enhancing OPH activity, the method including administering an extract of fenugreek to a living body. In an aspect, the present disclosure relates to a method of enhancing OPH activity, the method including administering an extract(s) of one or more types of plants selected from the group consisting of fenugreek, fennel, and hibiscus to a living body. In an aspect, the present disclosure relates to a method of enhancing OPH activity, the method including allowing a human or a non-human organism to take in an extract(s) of one or more types of plants selected from the group consisting of fenugreek, fennel, and hibiscus. In one or more embodiments, the method of enhancing OPH activity of the present disclosure may use, instead of the above-mentioned extract(s), an OPH activity enhancing agent, a beverage composition, a food composition, a functional food, a cosmetic, a supplement, a quasi drug, or a pharmaceutical product of the present disclosure.

[Method of producing beverage composition, food composition, functional food, cosmetic, or supplement for enhancing OPH activity]

In another aspect, the present disclosure relates to a method of producing a beverage composition, a food composition, a functional food, a cosmetic, or a supplement (a method of producing a beverage composition, etc. of the present disclosure). The method of producing a beverage composition, etc. of the present disclosure includes adding an extract of fenugreek to a beverage composition, a food composition, a functional food, a cosmetic, or a supplement in order to provide it with an OPH activity enhancing effect. The method of producing a beverage composition, etc. of the present disclosure includes adding an extract(s) of one or more types of plants selected from the group consisting of fenugreek, fennel, and hibiscus to a beverage composition, a food composition, a functional food, a cosmetic, or a supplement in order to provide it with an OPH activity enhancing effect.

In one or more embodiments, examples of the extract include a water extract and an organic solvent extract of each plant mentioned above. In one or more embodiments, examples of the water extract include a hot water extract. In one or more embodiments, examples of the form of the extract include an aqueous solution, a concentrated liquid, and a dried product.

[Extract composition for enhancing OPH activity]

In another aspect, the present disclosure relates to an extract composition of one or more types of plants selected from the group consisting of fenugreek, fennel, and hibiscus, the extract composition being for enhancing oxidized protein hydrolase activity. In the present disclosure, the term "extract composition" refers to a liquid or a solid that is obtained by an extraction treatment of a plant. In one or more embodiments, examples of the extract composition include a supernatant and a filtrate that are obtained by solvent-extracting a plant. In one or more embodiments, examples of the extractant (solvent) include water, alcohols such as methanol and ethanol, and ether. In one or more embodiments, examples of the form of the extract composition include an aqueous solution, a concentrated liquid, and a dried product. In one or more embodiments, the extract composition of the present disclosure can be used as a food raw material. In the present disclosure, the term "food raw material" refers to an ingredient or a raw material that is used for producing, for example, foods such as processed foods and health foods as well as supplements. In one or more embodiments, examples of the form of the food raw material include an aqueous solution, a concentrated liquid, and a dried product.

[Glycation stress inhibitor]

The present invention is based on a new finding that a mixture of water extracts, particularly hot water extracts, of fenugreek (scientific name: *Trigonella foenum -graecum)* and fennel (scientific name: *Foeniculum vulgare)* has an effect of activating oxidized protein hydrolase (OPH) known to preferentially decompose substances resulting from oxidative stress and glycation stress in a living body, such as oxidized proteins and glycated proteins, and has a higher ability to decompose AGEs than that of PTB known as an AGEs decomposing agent.

The present invention is based on a new finding that a water extract, particularly a hot water extract, of fenugreek has amylase and $\alpha$-glucosidase inhibitory activities. Furthermore, the present invention is based on a new finding that the use of a combination of an extract of fenugreek and an extract of fennel allows abnormal proteins to be reduced in one or more embodiments. The present disclosure is based on a new finding that the use of a combination of an extract of fenugreek and an extract of fennel allows glycation stress to be inhibited pleiotropically in one or more embodiments.

The glycation stress is a concept that comprehensively includes the stress put on a living body by reducing sugar or aldehyde stress and the reaction thereafter. In the present invention, the expression "inhibiting glycation stress" includes inhibiting the production of abnormal proteins, decomposing and/or removing abnormal proteins, and inhibiting hyperglycemia. Furthermore, in one or more embodiments, examples of inhibiting glycation stress may include inhibiting the production of oxidized substances that cause the production of abnormal proteins, or decomposing or eliminating oxidized substances.

The term "abnormal proteins" refers to proteins subjected to, for example, oxidation, glycation, or aldehyde modification. In one or more embodiments, examples of the abnormal proteins include products produced by the Maillard reaction (Maillard reaction products). In one or more embodiments, examples of the Maillard reaction products include intermediates produced by the Maillard reaction and final products produced by the Maillard reaction. In one or more embodiments, examples of the Maillard reaction products include polypeptides and proteins containing AGEs, polypeptides and proteins having cross-linked structures formed by AGEs, and glycated proteins produced by the Maillard reaction. In one or more embodiments, examples of the abnormal proteins include polypeptides and proteins with Schiff base/with Schiff base formed therein, Amadori compounds (ketoamine, glycated proteins), polypeptides and proteins each containing an $\alpha$-dicarbonyl compound, and polypeptides and proteins containing AGEs.

In one or more embodiments, examples of inhibiting the production of abnormal proteins include inhibiting the production of substances involved directly or indirectly in the production of abnormal proteins. In one or more embodiments, examples of inhibiting the production of said substances include inhibiting amylase and inhibiting $\alpha$-glucosidase. In one or more embodiments, examples of the substances involved directly or indirectly in the production of abnormal proteins include reducing sugars, $\alpha$-dicarbonyl compounds such as glyoxal, methylglyoxal, and glyceraldehyde, compounds with Schiff base, Amadori compounds, aldehydes, and AGEs. In one or more embodiments, examples of AGEs include amino acids and dipeptides glycated by the Maillard reaction and non-limiting examples thereof include pentosidine, crossline, pyrropyridine, pyrraline, carboxymethyllysine (CML), carboxyethyllysine, carboxymethylarginine (CMA), argpyrimidine, an imidazolone compound, glyoxal-lysine dimer (GOLD), and methyl glyoxal-lysine dimer (MOLD).

In one or more embodiments, examples of decomposing and/or removing abnormal proteins include decomposing Maillard reaction products. In one or more embodiments, examples of decomposing Maillard reaction products include breaking cross-linked structures formed between polypeptides and/or proteins by AGEs produced upon the Maillard reaction. In one or more embodiments, examples of decomposing Maillard reaction products may further include decomposing polypeptides and proteins that contain AGEs, polypeptides and proteins having cross-linked structures formed by AGEs, or glycated proteins produced by the Maillard reaction. Furthermore, in one or more embodiments that are not particularly limited, examples of decomposing Maillard reaction products may include breaking (cleaving) the C-C bonds in diketone structures contained in polypeptides and proteins that contain AGEs, polypeptides and proteins having cross-linked structures formed by AGEs, or glycated proteins produced by the Maillard reaction.

In one or more embodiments, examples of inhibiting a rapid increase in blood glucose level include inhibiting amylase, inhibiting $\alpha$-glucosidase, and inhibiting the production of reducing sugar by, for example, inhibitions thereof. In one or more embodiments, examples of the reducing sugar include monosaccharides such as glucose and fructose as well as maltose-type disaccharides/oligosaccharides such as lactose and maltose.

In one or more embodiments, examples of inhibiting hyperglycemia may include inhibiting a rapid increase in blood glucose level.

Examples of inhibiting oxidative stress include using a substance containing a component with a high anti-oxidative activity.

In an aspect, the present invention relates to non-therapeutic use of a glycation stress inhibitor containing extracts of fenugreek and fennel as active ingredients (a glycation stress inhibitor of the present invention) as defined in the claims.

The glycation stress inhibitor of the present invention refers to one having at least a Maillard reaction product decomposition activity (also referred to as "AGEs decomposition activity") and an OPH activity enhancing ability. In one or more embodiments, the glycation stress inhibitor of the present invention may have, as a further function(s), one or more of the functions such as an amylase inhibitory action, an $\alpha$-glucosidase inhibitory action, and an antioxidant activity. Furthermore, in one or more embodiments, the glycation stress inhibitor of the present invention can perform at least one of inhibition of production, decomposition, or removal of substances that cause abnormal protein production, or decomposition, removal, or inhibition of production of abnormal proteins.

The glycation stress inhibitor of the present invention contains extracts of fenugreek and fennel as active ingredients. Therefore, in one or more embodiments, the glycation stress inhibitor of the present invention enhances OPH enzyme activity and break cross-links to improve efficiency of decomposing oxidized proteins and glycated proteins. In one or more embodiments, the glycation stress inhibitor of the present invention can promote decomposition of cross-linked structures formed between polypeptides and/or proteins by AGEs produced in a living body to inhibit the accumulation of AGEs. In one or more embodiments, the glycation stress inhibitor of the present invention has an amylase or $\alpha$-glucosidase inhibitory action and therefore can inhibit a rapid increase in blood glucose level to inhibit, for example, a nonenzymatic reaction between reducing sugar and protein and a poor reaction in the TCA cycle (the tricarboxylic acid cycle) associated with the presence of excessive glucose, and as a result, it can inhibit the production of abnormal proteins. Furthermore, since the glycation stress inhibitor of the present invention has an antioxidant action, it can pleiotropically inhibit the production and accumulation of various abnormal proteins associated with glycation stress and oxidative stress. Hence, in one or more embodiments, the glycation stress inhibitor of the present invention allows glycation stress and/or oxidative stress to be pleiotropically inhibited or reduced. Moreover, in one or more embodiments, the glycation stress inhibitor of the present invention can be expected to have an anti-aging effect, an aging preventing effect, or an effect of preventing lifestyle-related diseases. Thus, in one or more embodiments, the glycation stress inhibitor of the present invention can also be said to be an anti-oxidative stress agent, an anti-aging agent, an aging preventing agent, or a lifestyle-related disease preventing agent.

Since the glycation stress inhibitor of the present invention contains extracts derived from plants such as fenugreek and fennel that are used as active ingredients, it can provide effects of excellent safety and excellent productivity.

In the glycation stress inhibitor of the present invention, the extract of fenugreek and the extract of fennel may be blended at the same ratio (1 part by weight of the extract of fennel with respect to 1 part by weight of the extract of fenugreek) or may be blended at different ratios.

In one or more embodiments, the blending ratio (the dry weight ratio) of the extract of fenugreek and the extract of fennel in the glycation stress inhibitor of the present invention is, per 1 part by weight of the extract of fenugreek, 0.1 to 10 parts by weight of the extract of fennel, and 0.2 to 5 parts by weight of the extract of fennel in terms of further improving the glycation stress inhibition effect.

In one or more embodiments, the glycation stress inhibitor of the present invention may contain an extract of hibiscus (scientific name: *Hibiscus sabdariffa)* as a further active ingredient in terms of further improving the antioxidative activity and the amylase inhibitory action. In one or more embodiments, the glycation stress inhibitor of the present invention contains extracts of fenugreek, fennel, and hibiscus as active ingredients.

When the glycation stress inhibitor of the present invention further contains an extract of hibiscus, the extract of hibiscus may be blended at the same ratio (1 part by weight with respect to 1 part by weight of the extract of fenugreek or the extract of fennel) as or at a different ratio from that of the extract of fenugreek and/or the extract of fennel.

In one or more embodiments, the glycation stress inhibitor of the present invention contains an extract of fenugreek, an extract of fennel, and an extract of hibiscus. In the present aspect, the blending ratio (the dry weight ratio) of the extract of fenugreek, the extract of fennel, and the extract of hibiscus is, per 1 part by weight of the extract of fenugreek, 0.05 to 20 parts by weight of the extract of fennel and 0.05 to 20 parts by weight of the extract of hibiscus, and 0.1 to 10 parts by weight of the extract of fennel and 0.1 to 10 parts by weight of the extract of hibiscus in terms of further improving the antioxidative activity and the amylase inhibitory action.

In one or more embodiments, the glycation stress inhibitor of the present invention may contain, as a further active ingredient, at least one of lemon balm (scientific name: *Melissa officinalis*) and rosemary (scientific name: *Rosmarinus officinalis*) in terms of further improving the ability to decompose AGEs.

The extracts may be extracted from any parts of plants. In one or more embodiments, examples of the part to be used include whole plants, flowers, leaves, seeds, fruits, branches, stems, roots, calyxes, and bracts. The part to be used may be of one type, or two or more types of parts may be combined for use. In one or more embodiments, fennel is preferably extracted from seeds. In one or more embodiments, fenugreek is preferably extracted from seeds. In one or more embodiments, hibiscus is preferably extracted from calyxes and bracts. In one or more embodiments, rosemary is preferably extracted from leaves. In one or more embodiments, lemon balm is preferably extracted from leaves.

The extraction method is not particularly limited, but in one or more embodiments, examples thereof include solvent extraction. In one or more embodiments, examples of the extractant (solvent) include water, alcohols such as methanol and ethanol, and ether. In one or more embodiments, examples of the extract include a water extract and an organic solvent extract. In one or more embodiments, examples of the water extract include a hot water extract. In one embodiment, the solvent extracting is performed at a temperature of 60-120°C, preferably 70-90°C such as 80°C or 100°C, for a time period of 15 minutes to 5 hours, preferably 2-6 hours or 3-5 hours such as 30 minutes or 1 hour. In a preferred embodiment, hot water extraction is carried out at a temperature of 60-120°C, preferably 70-90°C such as 80°C or 100°C, for a time period of 30 minutes to 5 hours, preferably 2-6 hours or 3-5 hours such as 30 minutes or 1 hour. In one or more embodiments, examples of the form of the extract include an aqueous solution, a concentrated liquid, and a dried product. Drying is carried out by heat-treating the extract obtained by the solvent-extracting at a temperature of 70-150°C such as 110°C for 1-10 hours such as 4 hours. A suitable method for producing a glycation stress inhibitor according to the present invention is as follows: A defined amount of dry powder of fenugreek (such as 2 g) and a defined amount of dry powder of fennel (such as 2 g) are extracted with a suitable amount of distilled water (such as 40 mL) at a temperature of 80°C for one hour, followed by cooling to normal temperature. Thereafter, the obtained slurry is filtrated and the filtrate thus obtained is used as a plant extract. If a dried plant extract shall be used, the obtained plant extract is put into an aluminum tray and dried for e.g. four hours in an incubator set at e.g. 110°C.

The form of the glycation stress inhibitor of the present invention is not particularly limited, but in one or more embodiments, examples thereof include solids, granules, powders, pastes, and liquids.

In one or more embodiments, the glycation stress inhibitor of the present invention may contain other extracts and other components as long as they do not inhibit the glycation stress inhibitory functions of the active ingredients.

In one or more embodiments, the glycation stress inhibitor of the present invention can be non-therapeutically used as a raw material for a beverage composition, a food composition, a functional food, a cosmetic, or a supplement. The form of the raw material is not particularly limited, but in one or more embodiments, examples thereof include solids, granules, powders, pastes, and liquids.

As one or more embodiments, the present invention relates to the non-therapeutic use of a glycation stress inhibitor, wherein the glycation stress inhibitor is comprised as an active agent in a beverage composition, a food composition, a functional food, a cosmetic, or a supplement.

The intake per day of the glycation stress inhibitor of the present invention is not particularly limited, but in one or more embodiments, it is at least 5 mg or not more than 2000 mg.

[Method of producing glycation stress inhibitor]

In another aspect, the present disclosure relates to a method of producing a glycation stress inhibitor, the method including solvent-extracting fenugreek and fennel. According to the production method of the present disclosure, the glycation stress inhibitor of the present disclosure can be produced.

In one or more embodiments, the production method of the present disclosure may include mixing an extract of fenugreek and an extract of fennel to obtain a mixture of the extracts. In one or more embodiments, the mixing ratios thereof may be the same (1 part by weight of the extract of fennel with respect to 1 part by weight of the extract of fenugreek) or may be different from each other.

In one or more embodiments, the production method of the present disclosure may include mixing an extract of fenugreek and an extract of fennel so that the extract of fennel is 0.1 to 10 parts by weight per 1 part by weight of the extract of fenugreek, and in terms of producing a glycation stress inhibitor that can further improve the glycation stress inhibition effect, the production method of the present disclosure may include mixing these extracts so that the extract of fennel is 0.2 to 5 parts by weight per 1 part by weight of the extract of fenugreek.

In one or more embodiments, the production method of the present disclosure may include mixing fenugreek and fennel and solvent-extracting the resultant mixture to obtain a mixed extract. In one or more embodiments, the mixing ratios thereof may be the same (1 part by weight of the fennel with respect to 1 part by weight of the fenugreek) or may be different from each other. Furthermore, they may be mixed together so that the ratios of the extracts of the respective plants in the resultant mixed extract are the same (1 part by weight of the extract of fennel with respect to 1 part by weight of the extract of fenugreek) or they may be mixed together so that the ratios thereof are different from each other.

In one or more embodiments, the method of producing a glycation stress inhibitor of the present disclosure may further include solvent-extracting at least one plant selected from the group consisting of hibiscus, lemon balm, and rosemary.

In one or more embodiments, the production method of the present disclosure may include solvent-extracting a dried plant.

In one or more embodiments, the production method of the present disclosure may include filtrating or centrifuging an extract obtained by solvent-extracting.

In one or more embodiments, the production method of the present disclosure may include drying a resultant extract.

In one or more embodiments, the production method of the present disclosure may include mixing an extract of fenugreek, an extract of fennel, and an extract of a plant other than these to obtain a mixture of the extracts. In one or more embodiments, the mixing ratio of the plant may be the same as that of the extract of fenugreek and/or the extract of fennel (1 part by weight with respect to 1 part by weight of the extract of fenugreek or the extract of fennel) or may be different.

In one or more embodiments, the production method of the present disclosure may include mixing an extract of fenugreek, an extract of fennel, and an extract of hibiscus so that per 1 part by weight of the extract of fenugreek, the extract of fennel is 0.05 to 20 parts by weight and the extract of hibiscus is 0.05 to 20 parts by weight, and in terms of producing a glycation stress inhibitor that can further improve the antioxidative activity and the amylase inhibitory action, the production method of the present disclosure may include mixing these extracts so that per 1 part by weight of the extract of fenugreek, the extract of fennel is 0.1 to 10 parts by weight and the extract of hibiscus is 0.1 to 10 parts by weight.

In one or more embodiments, the production method of the present disclosure may include mixing fenugreek, fennel, and a plant other than these and solvent-extracting the resultant mixture to obtain a mixed extract. In one or more embodiments, the mixing ratio of the plant may be the same as that of the fenugreek and/or the fennel (1 part by weight with respect to 1 part by weight of the fenugreek or the fennel) or may be different from them. Furthermore, they may be mixed so that the ratios of the extracts of the respective plants in the resultant mixed extract are the same (1 part by weight with respect to 1 part by weight of the extract of fenugreek or the extract of fennel) or different from each other.

[Method of producing beverage composition, food composition, functional food, cosmetic, or supplementfor providing it with glycation stress inhibitory function]

In another aspect, the present disclosure relates to a method of producing a beverage composition, a food composition, a functional food, a cosmetic, or a supplement(a second method of producing a beverage composition, etc. of the present disclosure). The second method of producing a beverage composition, etc. of the present disclosure includes adding extracts of fenugreek and fennel to a beverage composition, a food composition, a functional food, a cosmetic, or a supplement in order to provide it with a glycation stress inhibition effect.

In one or more embodiments, the second method of producing a beverage composition, etc. of the present disclosure may further include adding an extract of at least one plant selected from the group consisting of hibiscus, lemon balm, and rosemary to a beverage composition, a food composition, a functional food, a cosmetic, or a supplement.

In one or more embodiments, examples of the extract include a water extract and an organic solvent extract of each plant mentioned above. In one or more embodiments, examples of the water extract include a hot water extract. In one or more embodiments, examples of the form of the extract include an aqueous solution, a concentrated liquid, and a dried product.

[Extract composition for providing glycation stress inhibitory function]

In another aspect, the present disclosure relates to an extract composition of fenugreek and fennel for providing a glycation stress inhibitory function. In one or more embodiments, examples of the extract composition include a supernatant and a filtrate that are obtained by solvent-extracting a plant. In one or more embodiments, examples of the extractant (solvent) include water, alcohols such as methanol and ethanol, and ether. In one or more embodiments, examples of the form of the extract composition include an aqueous solution, a concentrated liquid, and a dried product. In one or more embodiments, the extract composition of the present disclosure can be used as a food raw material. In one or more embodiments, examples of the form of the food raw material include an aqueous solution, a concentrated liquid, and a dried product.

In one or more embodiments, the extract composition of the present disclosure may contain an extract of at least one plant selected from the group consisting of hibiscus, lemon balm, and rosemary.

Hereinafter, the present disclosure is further described using examples.

Examples

[Preparation of plant extracts]

After 2 g of dry powder of each plant indicated in Table 1 below was extracted with 40 ml of distilled water at 80°C for one hour, it was cooled to a normal temperature. Thereafter, it was filtrated and the filtrate thus obtained was used as a plant extract.

After 5 mL of resultant filtrate was put into an aluminum tray and then was dried for four hours in an incubator set at 110°C, the remaining amount thereof was measured to calculate the solid content concentration of each sample. The results are indicated in Table 1 below.

[OPH activation confirmatory test (Test 1)]

Acylamino-acid releasing enzyme (AARE) (manufactured by Takara Bio Inc.) used as OPH was dissolved in a sodium phosphate buffer solution provided with it, which then was adjusted to be 0.5 U/ml. Thereafter, this was diluted 500 times with 0.2 mol/L of Tris-HCl (pH 7.4) for use. For the reaction substrate for the OPH, 0.05 mol/L of N-acetyl-L-alanine p-nitroanilide (AAPA) 50% ethanol solution was used.

The extracts of the plants indicated in Table 1 below, the OPH Solution, the AAPA solution, and Tris-HCl were added to a 96-well microplate (Perkin Elmer) so as to be mixed in the blending amounts indicated in Table 2 below. The microplate was placed in a microplate reader (SpectraMax Paradigm Multi-Mode Microplate Reader) (manufactured by Molecular Devise) having a temperature adjusted to 37°C to allow them to react for 48 hours (for 2880 minutes) and then the absorbance at a wavelength of 405 nm was measured successively.

The OPH activity was evaluated based on the result of the 24-hour reaction. In terms of the relative values obtained when the OPH activity of decomposing the AAPA (no plant extract added) was taken as 100%, the absorbance of each of the reaction solutions A, B, C, and D was used to calculate the OPH activation rate (%) of each plant extract from the following formula. The results are indicated in Table 1 below as the relative values obtained when the activation rate of lemon balm is taken as 100.

$$\text{OPH activation rate (\%)} = (A-B) / (C-D) \times 100$$

Table 1

| | Common Name (Part to be Used) | Solid Content Concentration (mg/mL) | OPH Activation Rate (Relative Value) |
|---|---|---|---|
| Example | Fennel (Seeds) | 11.509 | 575 |
| | Hibiscus (Calyxes, Bracts) | 22.079 | 516 |
| | Fenugreek (Seeds) | 10.955 | 655 |
| Comp. Example | Thyme | 14.544 | 119 |
| | Rosemary | 11.107 | 120 |
| | Lemon Balm | 13.229 | 100 |

Table 2

| | Reaction Solution (μL) | | | |
|---|---|---|---|---|
| | A | B | C | D |
| Plant Extract | 10 | 10 | 0 | 0 |
| OPH Solution (0.5 U/ml) | 10 | 0 | 10 | 0 |
| Substrate (0.05 mol/L AAPA Solution) | 20 | 20 | 20 | 20 |
| 0.2 mol/L Tris-HCl (pH 7.4) | 210 | 220 | 220 | 230 |

As indicated in Table 1, fennel, hibiscus, and fenugreek each exhibited an OPH activity increasing effect that is at least 5 times that of lemon balm.

[Preparation of plant extract powder]

To each plant indicated in Table 3 below, 15 times its amount of water was added and it was extracted at 100°C for 30 minutes and then was solid-liquid separated and condensed. Thereafter, it was sterilized at 100°C or higher for 30 minutes or less, which then was spray-dried. Thus, plant extract powders were obtained.

[Preparation of sample solution]

Each plant extract powder mentioned above was diluted with distilled water so as to be 5 mg/ml, which then was used as a sample solution.

[OPH activation confirmatory test (Test 2)]

Acylamino-acid releasing enzyme (AARE) (manufactured by Takara Bio Inc.) was dissolved in a sodium phosphate buffer solution provided with it and then 0.2mol/L of Tris-HCl (pH 7.4) was used to prepare 0.01 U/ml, 0.005 U/ml, or 0.001 U/ml of OPH solution. For the reaction substrate for the OPH, 0.05 mol/L of N-acetyl-L-alanine p-nitroanilide (AAPA) 50% ethanol solution was used.

The OPH Solution, the AAPA solution, and the sample solution (5 mg/ml) were mixed together to be added to each well of a 96-well microplate and then were reacted for 24 hours in an incubator set at 37°C. The absorbance at 405 nm of each reaction solution was measured with a microplate reader.

The enzyme activity of the OPH was determined as variation in absorbance (reaction rate) per hour (the OPH reaction rate of the sample). On the other hand, as a reference (Ref), the same measurement was carried out without adding the sample solution and then the reaction rate was determined (the OPH reaction rate of Ref). Using the OPH reaction rate of the sample and the OPH reaction rate of Ref, the OPH activation rate (%) was calculated from the following formula (n=3). The results are indicated in Table 3 below.

OPH activation rate (%) = {(OPH reaction rate of sample) / (OPH reaction rate of Ref)} × 100

Using Epigallocatechin gallate (EGCg) (5 mg/ml) as a negative control, the same measurement was carried out. The results are indicated in Table 3 below.

Table 3

| | Blending Ratio (Weight Ratio) | OPH Activation Rate (%) (Mean Value ± Standard Deviation) |
|---|---|---|
| Fennel Extract Powder | - | 188.1 ± 3.0 |
| Hibiscus Extract Powder | - | 170.2 ± 5.1 |
| Fenugreek Extract Powder | - | 189.1 ± 2.4 |
| Fennel : Hibiscus : Fenugreek | 1:1:1 [*1] | 193.0 ± 6.5 |
| | 4:1:2 [*2] | 198.7 ± 5.7 |
| | 4:1:4 [*3] | 181.2 ± 1.9 |
| | 7:1:7 [*4] | 183.1 ± 1.6 |
| EGCg (Negative Control) | | 15.5 ± 3.2 |

[*1] per 1 part by weight of the fenugreek extract powder, 1 part by weight of the fennel extract powder, and 1 part by weight of the hibiscus extract powder
[*2] per 1 part by weight of the fenugreek extract powder, 2 parts by weight of the fennel extract powder, and 0.5 parts by weight of the hibiscus extract powder
[*3] per 1 part by weight of the fenugreek extract powder, 1 part by weight of the fennel extract powder, and 0.25 parts by weight of the hibiscus extract powder
[*4] per 1 part by weight of the fenugreek extract powder, 1 part by weight of the fennel extract powder, and 0.14 parts by weight of the hibiscus extract powder

As shown in Table 3, an OPH activity increasing effect of higher than 170% was confirmed in each case. Furthermore, even when fennel, hibiscus, and fenugreek were used in combination, OPH activity increasing effects that were comparable with or higher than those obtained when they were used individually were confirmed.

[AGEs cross-link breaking effect confirmatory test (Test 1)]

The test was carried out according to the description in Sara Vasan et al. (Nature, 382, pp275-278 (1996)).

As a substrate, 1-phenyl-1,2-propanedione (PPD), which is a reaction substrate for an AGEs cross-linking model having an $\alpha$-diketone structure, was used while N-phenacylthiazolium bromide (PTB) was used as a positive control.

A plant extract, 10 mmol/L of PPD, and 0.2 mol/L of phosphate buffer solution (pH 7.4) were mixed together at a ratio of 5:1:4 and then were reacted at 37°C for eight hours (n=3). After completion of the reaction, hydrochloric acid was added thereto to stop the reaction and thus a reaction solution was obtained.

The reaction solution was centrifuged at 20 °C and 3,000 × g for ten minutes and then the amount of benzoic acid contained in the supernatant was analyzed by reverse-phase HPLC. The amount of benzoic acid contained in the reaction solution was determined by subtracting the amount of benzoic acid contained in the plant extract, which was measured separately. Since 1 mol of PPD produces 1 mol of benzoic acid, the cross-link breaking rate was calculated by the following formula. The results are indicated in Table 4 below.

$$\text{Cross-link breaking rate (\%)} = \{(A\text{-}B) / C\} \times 100$$

A: The amount of benzoic acid contained in the reaction solution
B: The amount of benzoic acid contained in the plant extract
C: The amount of PPD provided for the reaction (the amount of the substrate)

As a control, a test was carried out using 10 mmol/L of PTB instead of the plant extract.

Table 4

| Common Name (Part to be Used) | Solid Content Concentration (mg/mL) | Cross-Link Breaking Rate (%) (Mean Value ± Standard Deviation) |
|---|---|---|
| Fennel (Seeds) | 1.15 | 39.00 ± 4.30 |
| Lemon Balm (Leaves) | 1.32 | 29.60 ± 2.14 |
| Rosemary (Leaves, Stems) | 1.11 | 26.58 ± 0.25 |
| PTB | - | 20.47 ± 0.29 |

As indicated in Table 4, fennel, lemon balm, and rosemary each exhibited a higher cross-link breaking rate than that of PTB that has been reported as a compound that breaks cross-linked structures associated with AGEs. Particularly, fennel exhibited a cross-link breaking rate nearly twice as high as that of PTB.

[AGEs cross-link breaking effect confirmatory test (Test 2)]

The test was carried out according to the description in Sara Vasan et al. (Nature, 382, pp275-278 (1996)).

300 µL/well of SuperBlock T20 (PBS) Blocking Buffer was added to a commercially available 96-well microplate precoated with collagen, which then was reacted at 37°C for one hour. After the reaction, it was washed with 0.05% polyethylene (20) sorbitan monolaurate (Tween 20)/PBS(-), and then AGEs modified bovine serum albumin (AGE-BSA) was added to each well of the microplate, which then was subjected to a crosslinking reaction at 37°C for four hours. After the reaction, it was washed with Tween 20/PBS(-). Thereafter, a plant extract was added to each well and thereby it was subjected to a cross-link breaking (cleaving) reaction at 37°C for 18 hours. After that, it was washed with Tween 20/PBS(-) and a primary antibody (anti-albumin bovine serum rabbit-polyclonal) was added thereto, which then was reacted at room temperature for 30 minutes. Further, after it was washed with Tween 20/PBS(-), a secondary antibody (goat anti-rabbit IgG horseradish peroxidase (HRP) conjugate) was added thereto, which then was reacted at room temperature for 30 minutes. Thereafter, it was washed with Tween 20/PBS(-), and TMB One Component HRP Microwell Substrate was added thereto. Furthermore, a PEG6000 solution was added thereto, which then was reacted for 20 minutes. As a reaction stop solution, 1N HCl was added thereto, and the absorbance at 450 nm (dominant wavelength) / 630 nm (complementary wavelength) was measured with a microplate reader. The amount of AGE-BSA that remained was determined from a calibration curve in which the amount of AGE-BSA was varied. Furthermore, the AGE-BSA decomposition rate (Collagen cross-link decomposition rate)was determined using the following formula. The results are indicated in Table 5 below.

AGE-BSA decomposition rate = {(Amount of AGE-BSA added - Amount of remaining AGE-BSA) / Amount of AGE-BSA added} $\times$ 100

As a control, the same test was carried out using, instead of the plant extract, 5mmol/L of punicalagin that has been known to have a Maillard reaction activity-inhibiting effect.

Table 5

| Common Name (Part to be Used) | Amount of Remaining AGE-BSA (ng) | Collagen Cross-Link Decomposition Rate (%) |
|---|---|---|
| Fennel (Seeds) | 812.4 | 18.8 |
| Lemon Balm (Leaves) | 373 | 62.7 |
| Fenugreek (Seeds) | 818.8 | 18.1 |
| Punicalagin | 343.3 | 65.7 |

As shown in Table 5, it was confirmed that fennel, lemon balm, and fenugreek each have a collagen cross-link breaking activity. It was confirmed that in particular, lemon balm has a collagen cross-link breaking activity equivalent to that of punicalagin.

[AGEs cross-link breaking effect confirmatory test (Test 3)]

The same measurement as in the AGEs cross-link breaking effect confirmatory test (Test 1) was carried out except for using a mixture of plant extracts indicated in Table 6 below. The results are indicated in Table 6 below.

Table 6

| Common Name (Blending Ratio (Weight Ratio)) | Solid Content Concentration (mg/mL) | Cross-Link Breaking Rate (%) (Mean Value $\pm$ Standard Deviation) |
|---|---|---|
| Fennel : Hibiscus : Fenugreek (1:1:1) | 7.42 | 42.10 $\pm$ 1.97 |
| PTB | - | 25.34 $\pm$ 0.19 |

As indicated in Table 6, the mixture of fennel, hibiscus, and fenugreek exhibited an AGEs cross-link breaking rate nearly twice as high as that of PTB.

[Amylase inhibitory action confirmatory test]

An Enzy Chrom $\alpha$-Amylase Assay Kit (BioAssay System) and $\alpha$-Amylase from Porcine pancreas (Sigma-Aldrich) were used.
10 $\mu$L each of enzyme, Assay Buffer, and Glucose Standard that were provided in a kit were dispensed into each well of a microplate. Thereafter, 40 $\mu$L each of reaction solutions containing the plant extracts indicated in Table 7 below was dispensed into each well, which then was reacted for 15 minutes after being stirred. Thereafter, 150 $\mu$L of color reagent was dispensed into each well, which then was reacted for 20 minutes at room temperature. Then, the absorbance at 585 nm thereof was measured and the inhibition rate and IC50 were determined (n=3). The IC50 thus obtained is indicated in Table 7 below.

Table 7

| Common Name (Part to be Used) | IC50 (mg/mL) |
|---|---|
| Fenugreek (Seeds) | 0.012 |
| Hibiscus (Calyxes, Bracts) | 0.097 |

As shown in Table 7, each IC50 of fenugreek and hibiscus was less than 0.1 mg/mL. Thus, it was confirmed that fenugreek and hibiscus each have a high inhibitory activity for amylase. This suggested that fenugreek and hibiscus can inhibit a rapid increase in blood glucose level and as a result, can inhibit the production of, for example, dicarbonyl compounds and AGEs that may cause abnormal proteins to be produced.

[a-Glucosidase inhibitory action confirmatory test]

QuantiChrom $\alpha$-Glucosidase Assay Kit DAGD-100 (BioAssay Systems) and rat small intestinal acetone powder (Sigma-Aldrich, $\alpha$-Glucosidase) were used.

After the temperature of the measurement part of a microplate reader was set at 30°C, 200 $\mu$L of sample solution (containing 8 uL of substrate solution) containing a plant extract indicated in Table 8 below and 20 $\mu$L of $\alpha$-glucosidase solution were dispensed into each well of a microplate and then were stirred to start a reaction. Then, the change in absorbance at 405 nm was measured for 30 minutes. An $\alpha$-glucosidase inhibitor, acarbose, was used as a positive control for the inhibitory action, and the $\alpha$-glucosidase activity inhibition rate (%) was calculated according to the following formula (n=3). Thus, IC50 was determined.

$$\alpha\text{-Glucosidase activity inhibition rate } (\%) = \{1 - (A / B)\} \times 100$$

A: Value of each concentration of sample and positive control ($\Delta$Abs 30 min)
B: TOTAL (all colours) value ($\Delta$Abs 30 min)

Table 8

| Common Name (Part to be Used) | IC50 (mg/mL) |
|---|---|
| Fenugreek (Seeds) | 1.550 |
| Fennel (Seeds) | 3.807 |

As shown in Table 8, it was confirmed that fenugreek and fennel each have an $\alpha$-glucosidase inhibitory activity. This suggested that fenugreek and fennel can inhibit a rapid increase in blood glucose level and as a result, can inhibit the production of, for example, dicarbonyl compounds and AGEs that may cause abnormal proteins to be produced.

[Antioxidative activity confirmatory test]

SPOTCHEM (Trademark) i-Pack Oxystress Test (Trademark) of ARKRAY, Inc. was used.
90 $\mu$L of SPOTCHEM reagent r1 containing thiocyanate and 36 $\mu$L of SPOTCHEM reagent r2 containing iron(III) chloride were mixed together. To 126 $\mu$L of this mixture, 18 $\mu$L of plant extract indicated in Table 9 below was added, and the absorbance at 465 nm was measured using SPOTCHEM. Thus, antioxidative activity ($\mu$mol/L) was determined.
On the other hand, instead of the plant extract, a standard reagent, ascorbic acid, was used and the absorbance was measured in the same manner. Then, a calibration curve that indicates the correlation between the ascorbic acid concentration and the absorbance was created. Then, using the absorbance of the plant extract and the calibration curve, the antioxidative activity was converted into the ascorbic acid equivalent (mg/L). The results are indicated in Table 9 below.

Table 9

| Common Name (Part to be Used) | Antioxidative Activity (AP) (mg Vitamin C Equivalent/L) |
|---|---|
| Fennel (Seeds) | 420 |
| Hibiscus (Calyxes, Bracts) | 3210 |
| Fenugreek (Seeds) | 271 |

As indicated in Table 9, it was confirmed that hibiscus has a high antioxidative activity. This suggested that hibiscus can eliminate oxidized substances that cause abnormal proteins to be produced.

**Claims**

1. Non-therapeutic use of an agent, comprising an extract of fenugreek as an active ingredient, for improving the efficiency of decomposing oxidized proteins and glycated proteins as a result of enhancing oxidized protein hydrolase activity, wherein the extract is a water extract.

2. The non-therapeutic use according to claim 1, wherein the oxidized protein hydrolase activity enhancing agent further comprises, as an active ingredient, an extract of at least one of fennel and hibiscus.

3. The non-therapeutic use according to any one of claims 1 to 2, wherein the oxidized protein hydrolase activity enhancing agent comprises extracts of fenugreek, fennel, and hibiscus as active ingredients.

4. The non-therapeutic use according to any one of claims 1 to 3, wherein the water extract is a hot water extract, preferably the extract obtainable by the method defined in claim 11.

5. The non-therapeutic use according to any one of claims 1 to 4, wherein the oxidized protein hydrolase activity enhancing agent comprises a dried product of the extract as an active ingredient.

6. Non-therapeutic use of a glycation stress inhibitor, comprising extracts of fenugreek and fennel as active ingredients, for decomposing oxidized proteins, glycated proteins or advanced glycation end products, wherein the glycation stress inhibitor has at least a Maillard reaction product decomposition activity and an oxidized protein hydrolase activity enhancing ability, wherein the extract(s) is/are water extract(s).

7. The non-therapeutic use according to claim 6, wherein the glycation stress inhibitor further comprises an extract of hibiscus as an active ingredient.

8. The non-therapeutic use according to claim 6 or 7, wherein the water extract(s) is/are hot water extract(s), preferably the extract(s) obtainable by the method defined in claim 11.

9. The non-therapeutic use according to any one of claims 6 to 8, wherein the glycation stress inhibitor comprises dried products of the extracts as active ingredients.

10. The non-therapeutic use according to claim 4 or claim 9, wherein

   (i) the oxidized protein hydrolase activity enhancing agent comprises an extract of fenugreek and an extract of hibiscus in a dry weight ratio of 0.1 to 10 parts by weight of the extract of hibiscus per 1 part by weight of the extract of fenugreek; or
   (ii) wherein the oxidized protein hydrolase activity enhancing agent or the glycation stress inhibitor comprises one of the following:

   (ii-1) an extract of fenugreek and an extract of fennel in a dry weight ratio of 0.1 to 10 parts by weight of the extract of fennel per 1 part by weight of the extract of fenugreek; or
   (ii-2) an extract of fenugreek, an extract of fennel, and an extract of hibiscus in a dry weight ratio of 0.05 to 20 parts by weight of the extract of fennel and 0.05 to 20 parts by weight of the extract of hibiscus per 1 part by weight of the extract of fenugreek.

11. The non-therapeutic use according to any one of claim 1 to 10, wherein at least one of the oxidized protein hydrolase activity enhancing agent and the glycation stress inhibitor is obtainable by a method comprising solvent-extracting fenugreek and optionally further comprising solvent-extracting at least one plant selected from the group consisting of fennel and hibiscus, wherein the solvent extracting is preferably performed at 60-120°C such as 80°C or 110°C for 30 minutes to 5 hours such as 30 minutes or 1 hour and wherein the extract obtained by the solvent-extracting is optionally dried e.g. at a temperature of 70-150°C such as 110°C for 1-10 hours such as 4 hours.

12. The non-therapeutic use according to any one of claim 1 to 10, wherein the oxidized protein hydrolase activity enhancing agent or the glycation stress inhibitor is comprised as an active agent in a beverage composition, a food composition, a functional food, a cosmetic or a supplement.

13. The non-therapeutic use according to claim 12, wherein the beverage composition, the food composition, the functional food, the cosmetic or the supplement is produced by a method comprising adding the extract(s) of fenugreek, fenugreek and fennel or hibiscus, or fenugreek, fennel and hibiscus to a beverage composition, a food composition, a functional food, a cosmetic or a supplement, in order to provide it with at least one of an oxidized protein hydrolase activity enhancing effect and a glycation stress inhibitory function.

**Patentansprüche**

1. Nicht-therapeutische Verwendung eines Mittels, umfassend einen Extrakt aus Bockshornklee als Wirkstoff, zur Verbesserung der Effizienz der Zersetzung oxidierter Proteine und glykierter Proteine durch Steigerung der oxidierten Proteinhydrolase-Aktivität, wobei der Extrakt ein Wasserextrakt ist.

2. Nicht-therapeutische Verwendung gemäß Anspruch 1, wobei das Mittel zur Steigerung der oxidierten Proteinhydrolase-Aktivität ferner umfasst, als Wirkstoff, einen Extrakt aus mindestens einem von Fenchel und Hibiskus.

3. Nicht-therapeutische Verwendung gemäß mindestens einem der Ansprüche 1 bis 2, wobei das Mittel zur Steigerung der oxidierten Proteinhydrolase-Aktivität umfasst Extrakte aus Bockshornklee, Fenchel und Hibiskus als Wirkstoffe.

4. Nicht-therapeutische Verwendung gemäß mindestens einem der Ansprüche 1 bis 3, wobei der Wasserextrakt ein Heißwasserextrakt ist, vorzugsweise der Extrakt erhältlich durch das in Anspruch 11 definierte Verfahren.

5. Nicht-therapeutische Verwendung gemäß mindestens einem der Ansprüche 1 bis 4, wobei das Mittel zur Steigerung der oxidierten Proteinhydrolase-Aktivität umfasst ein getrocknetes Produkt des Extrakts als Wirkstoff.

6. Nicht-therapeutische Verwendung eines Glykationsstress-Inhibitors, umfassend Extrakte aus Bockshornklee und Fenchel als Wirkstoffe, zur Zersetzung von oxidierten Proteinen, glykierten Proteinen oder fortgeschrittenen Glykationsendprodukten, wobei der Glykationsstress-Inhibitor mindestens eine Maillard-Reaktionsprodukt-Zersetzungsaktivität und eine oxidierte Proteinhydrolase-Aktivitätssteigerungsfähigkeit aufweist, wobei der/die Extrakt(e) Wasserextrakt(e) ist/sind.

7. Nicht-therapeutische Verwendung gemäß Anspruch 6, wobei der Glykationsstress-Inhibitor ferner umfasst einen Extrakt aus Hibiskus als Wirkstoff.

8. Nicht-therapeutische Verwendung gemäß Anspruch 6 oder 7, wobei der/die Wasserextrakt(e) Heißwasserextrakt(e) ist/sind, vorzugsweise der/die Extrakt(e), der/die durch das in Anspruch 11 definierte Verfahren erhältlich ist/sind.

9. Nicht-therapeutische Verwendung gemäß mindestens einem der Ansprüche 6 bis 8, wobei der Glykationsstress-Inhibitor umfasst getrocknete Produkte der Extrakte als Wirkstoffe.

10. Nicht-therapeutische Verwendung gemäß Anspruch 4 oder Anspruch 9, wobei

    (i) das Mittel zur Steigerung der oxidierten Proteinhydrolase-Aktivität umfasst einen Extrakt aus Bockshornklee und einen Extrakt aus Hibiskus in einem Trockengewichtsverhältnis von 0,1 bis 10 Gewichtsteilen des Extrakts aus Hibiskus pro 1 Gewichtsteil des Extrakts aus Bockshornklee; oder
    (ii) wobei das Mittel zur Steigerung der oxidierten Proteinhydrolase-Aktivität oder der Glykationsstress-Inhibitor umfasst eines der folgenden:

    (ii-1) einen Extrakt aus Bockshornklee und einen Extrakt aus Fenchel in einem Trockengewichtsverhältnis von 0,1 bis 10 Gewichtsteilen des Extrakts aus Fenchel pro 1 Gewichtsteil des Extrakts aus Bockshornklee; oder
    (ii-2) einen Extrakt aus Bockshornklee, einen Extrakt aus Fenchel und einen Extrakt aus Hibiskus in einem Trockengewichtsverhältnis von 0,05 bis 20 Gewichtsteilen des Extrakts aus Fenchel und 0,05 bis 20 Gewichtsteilen des Extrakts aus Hibiskus pro 1 Gewichtsteil des Extrakts aus Bockshornklee.

11. Nicht-therapeutische Verwendung gemäß mindestens einem der Ansprüche 1 bis 10, wobei mindestens eines aus dem Mittel zur Steigerung der oxidierten Proteinhydrolase-Aktivität und dem Glykationsstress-Inhibitor durch ein Verfahren erhältlich ist, umfassend Lösungsmittelextraktion von Bockshornklee und optional ferner umfassend Lösungsmittelextraktion mindestens einer Pflanze ausgewählt aus der Gruppe, bestehend aus Fenchel und Hibiskus, wobei die Lösungsmittelextraktion vorzugsweise bei 60-120 °C, beispielsweise 80 °C oder 110 °C, für 30 Minuten bis 5 Stunden, beispielsweise 30 Minuten oder 1 Stunde, durchgeführt wird und wobei der durch die Lösungsmittelextraktion erhaltene Extrakt optional getrocknet wird, beispielsweise bei einer Temperatur von 70 bis 150 °C, beispielsweise 110 °C, für 1 bis 10 Stunden, beispielsweise 4 Stunden.

12. Nicht-therapeutische Verwendung gemäß mindestens einem der Ansprüche 1 bis 10, wobei das Mittel zur Steigerung der oxidierten Proteinhydrolase-Aktivität oder der Glykationsstress-Inhibitor als Wirkstoff in einer Getränkemittelzusammensetzung, einer Lebensmittelzusammensetzung, einem funktionellen Lebensmittel, einem Kosmetikum oder einem Nahrungsergänzungsmittel enthalten ist.

**13.** Nicht-therapeutische Verwendung gemäß Anspruch 12, wobei die Getränkemittelzusammensetzung, die Nahrungsmittelzusammensetzung, das funktionelle Nahrungsmittel, das Kosmetikum oder das Nahrungsergänzungsmittel durch ein Verfahren hergestellt wird, umfassend das Hinzufügen des Extrakts/der Extrakte aus Bockshornklee, Bockshornklee und Fenchel oder Hibiskus, oder Bockshornklee, Fenchel und Hibiskus zu einer Getränkemittelzusammensetzung, einer Nahrungsmittelzusammensetzung, einem funktionellen Lebensmittel, einem Kosmetikum oder einem Nahrungsergänzungsmittel, um dieser mindestens eine aus einer steigernden oxidierte Proteinhydrolase-Aktivität und eine Glykationsstress-Inhibitor Funktion zu verleihen.

**Revendications**

**1.** Utilisation non thérapeutique d'un agent, comprenant un extrait de fenugrec comme principe actif, pour améliorer l'efficacité de la décomposition de protéines oxydées et de protéines glyquées du fait de l'augmentation de l'activité hydrolase des protéines oxydées, dans lequel l'extrait est un extrait d'eau.

**2.** Utilisation non thérapeutique selon la revendication 1, dans laquelle l'agent améliorant l'activité hydrolase des protéines oxydées comprend en outre, comme principe actif, un extrait d'au moins un parmi le fenouil et l'hibiscus.

**3.** Utilisation non thérapeutique selon l'une quelconque des revendications 1 à 2, dans laquelle l'agent améliorant l'activité hydrolase des protéines oxydées comprend des extraits de fenugrec, de fenouil et d'hibiscus comme principes actifs.

**4.** Utilisation non thérapeutique selon l'une quelconque des revendications 1 à 3, dans laquelle l'extrait d'eau est un extrait d'eau chaude, l'extrait pouvant de préférence être obtenu par le procédé défini dans la revendication 11.

**5.** Utilisation non thérapeutique selon l'une quelconque des revendications 1 à 4, dans laquelle l'agent améliorant l'activité hydrolase des protéines oxydées comprend un produit sec de l'extrait comme principe actif.

**6.** Utilisation non thérapeutique d'un inhibiteur du stress de glycation, comprenant des extraits de fenugrec et de fenouil comme principes actifs, pour décomposer des protéines oxydées, des protéines glyquées ou des produits finaux de glycation avancée, dans laquelle l'inhibiteur du stress de glycation présente au moins une activité de décomposition de produits issus de la réaction de Maillard et une capacité d'amélioration de l'activité hydrolase des protéines oxydées, dans laquelle l'extrait ou les extraits est/sont un ou des extrait(s) d'eau.

**7.** Utilisation non thérapeutique selon la revendication 6, dans laquelle l'inhibiteur du stress de glycation comprend en outre un extrait d'hibiscus comme principe actif.

**8.** Utilisation non thérapeutique selon la revendication 6 ou 7, dans laquelle l'extrait(s) d'eau est un extrait(s) d'eau chaude, l'extrait(s) pouvant de préférence être obtenu par le procédé défini dans la revendication 11.

**9.** Utilisation non thérapeutique selon l'une quelconque des revendications 6 à 8, dans laquelle l'inhibiteur du stress de glycation comprend des produits secs des extraits comme principes actifs.

**10.** Utilisation non thérapeutique selon la revendication 4 ou 9, dans laquelle

(i) l'agent améliorant l'activité hydrolase des protéines oxydées comprend un extrait de fenugrec et un extrait d'hibiscus dans un rapport en poids sec de 0,1 à 10 parties en poids de l'extrait d'hibiscus pour 1 partie en poids de l'extrait de fenugrec ; ou
(ii) dans laquelle l'agent améliorant l'activité hydrolase des protéines oxydées ou l'inhibiteur du stress de glycation comprend l'un des composés suivants :

(ii-1) un extrait de fenugrec et un extrait de fenouil dans un rapport en poids sec de 0,1 à 10 parties en poids de l'extrait de fenouil pour 1 partie en poids de l'extrait de fenugrec ; ou
(ii-2) un extrait de fenugrec, un extrait de fenouil et un extrait d'hibiscus dans un rapport en poids sec de 0,05 à 20 parties en poids de l'extrait de fenouil et de 0,05 à 20 parties en poids de l'extrait d'hibiscus pour 1 partie en poids de l'extrait de fenugrec.

11. Utilisation non thérapeutique selon l'une quelconque des revendications 1 à 10, dans laquelle au moins un parmi l'agent d'amélioration de l'activité hydrolase des protéines oxydées et l'inhibiteur du stress de glycation peut être obtenu par un procédé comprenant du fenugrec obtenu par extraction au solvant et comprenant en outre optionnellement une extraction au solvant d'au moins une plante sélectionnée parmi le groupe consistant en le fenouil et l'hibiscus, dans laquelle l'extraction au solvant est de préférence effectuée à une température comprise entre 60 et 120 °C, telle que 80 °C ou 110 °C, pendant 30 minutes à 5 heures, telle que 30 minutes ou 1 heure, et dans laquelle l'extrait obtenu par extraction au solvant est éventuellement séché, par exemple à une température comprise entre 70 et 150 °C, telle que 110 °C, pendant 1 à 10 heures, par exemple 4 heures.

12. Utilisation non thérapeutique selon l'une quelconque des revendications 1 à 10, dans laquelle l'agent améliorant l'activité hydrolase des protéines oxydées ou l'inhibiteur du stress de glycation est compris comme agent actif dans une composition de boisson, une composition alimentaire, un aliment fonctionnel, un cosmétique ou un complément alimentaire.

13. Utilisation non thérapeutique selon la revendication 12, dans laquelle la composition de boisson, la composition alimentaire, l'aliment fonctionnel, le cosmétique ou le complément alimentaire est produit par un procédé comprenant un ajout du ou des extrait(s) de fenugrec, de fenugrec et de fenouil ou d'hibiscus, ou de fenugrec, de fenouil et d'hibiscus à une composition de boisson, une composition alimentaire, un aliment fonctionnel, un cosmétique ou un complément alimentaire, afin de lui conférer au moins l'un des effets suivants : un effet améliorant l'activité hydrolase des protéines oxydées et une fonction inhibitrice du stress de glycation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011004733 A
- WO 2014126199 A
- JP 2007119373 A
- EP 2949362 A1

**Non-patent literature cited in the description**

- **SARA VASAN et al.** *Nature*, 1996, vol. 382, 275-278
- **SARA VASAN et al.** *Archives of Biochemistry and Biophysics*, 2003, vol. 419, 89-96